# EUROPEAN PATENT SPECIFICATION

(11) **EP 1 832 587 B1**
(45) Date of publication and mention of the grant of the patent: **22.04.2009**
(21) Application number: 07103405.2
(22) Date of filing: 02.03.2007
(51) Int. Cl.: C07D 471/04

(54) **Method for preparing moxifloxacin and moxifloxacin hydrochloride**
Herstellungsverfahren für Moxifloxacin und Moxifloxacinhydrochlorid
Procédé de préparation de moxifloxacine et d'hydrochlorure de moxifloxacine

(30) Priority: 10.03.2006 ES 200600608
(43) Date of publication of application: 12.09.2007
(73) Proprietor: Quimica Sintetica, S.A., 08028 Barcelona (ES)
(72) Inventor: Palomo Nicolau, Francisco, 28804, ALCALA DE HENARES (ES); Cosme Gomez, Antonio, 28813, TORRES DE LA ALAMEDA (ES); Villasante Prieto, Javier, 28007, MADRID (ES); Fernandez Llorca, Sara Penélope, 28803, ALCALA DE HENARES (ES); Molina Ponce, Andrés, 19200, ALCALA DE HENARES (ES)
(74) Representative: Ponti Sales, Adelaida

(56) References cited:
- EP-A- 0 550 903
- EP-A2- 0 610 958
- WO-A-20/04091619
- WO-A-20/05012285
- WO-A-20/05047260
- ES-A1- 2 077 490
- ES-A6- 2 017 864

## Description

### Field of the technique

The present invention relates to a "one pot" method for preparing moxifloxacin and its hydrochloride salt, without any need to isolate the intermediate compounds.

### Background of the invention

Moxifloxacin hydrochloride is the international nonproprietary name of 1-cyclopropyl-6-fluoro-1,4-dihydro-8-methoxy-7-[(4aS,7aS)-octahydro-6H-pyrrol[3,4-b]pyridin-6-il]-4-oxo-3-quinolin carboxylic hydrochloride acid of Formula (I), finding application in medicine and used as an antibacterial agent.

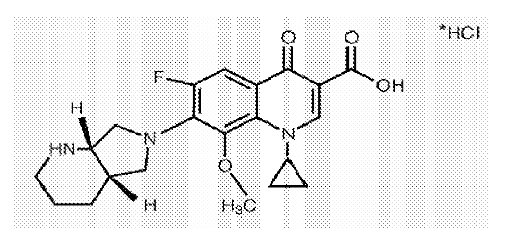

Recemic moxifloxacin was disclosed for the first time in European patent EP 350.733-B1. European application EP-A-550.903 claims moxifloxacin, a product of configuration (S,S), and its preparation by condensation of the compound (II) with (S,S)-2,8-diaza-bicyclo[4.3.0]nonane (III), optionally, in the presence of an acids captor. The compound (III) is prepared from 6-bencil-2,8-diaza-bicyclo[4.3.0]nonane: 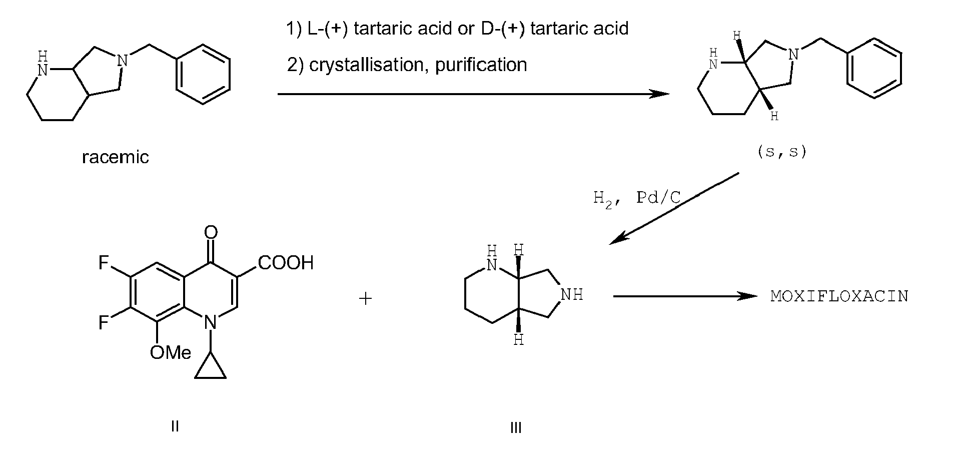 According to this process, the moxifloxacin of melting point 203-208°C is isolated by means of a chromatographic process. It has been found that the yield of the reaction varies between 57% and 80%, depending on the number of bicyclic amine equivalents used (1.05-4.2 equivalents, respectively), i.e., the larger the amount of bicyclic amine used the better the yield. It has also been found that there forms in the process between 10% and 20% of a undesirable by-product resulting from the demethylation in 8-position.

The main disadvantage of this method lies in the cost of the initial bicyclic amine, in that obtaining a good yield requires an excess of at least 4.2 equivalents thereof. Said method also produces as an impurity a by-product that is difficult to remove.

Application WO 2005/012285-A1 describes a new method for obtaining monohydrate moxifloxacin hydrochloride by using an intermediate of formula IV without the additional stage of isolating the base moxifloxacin.

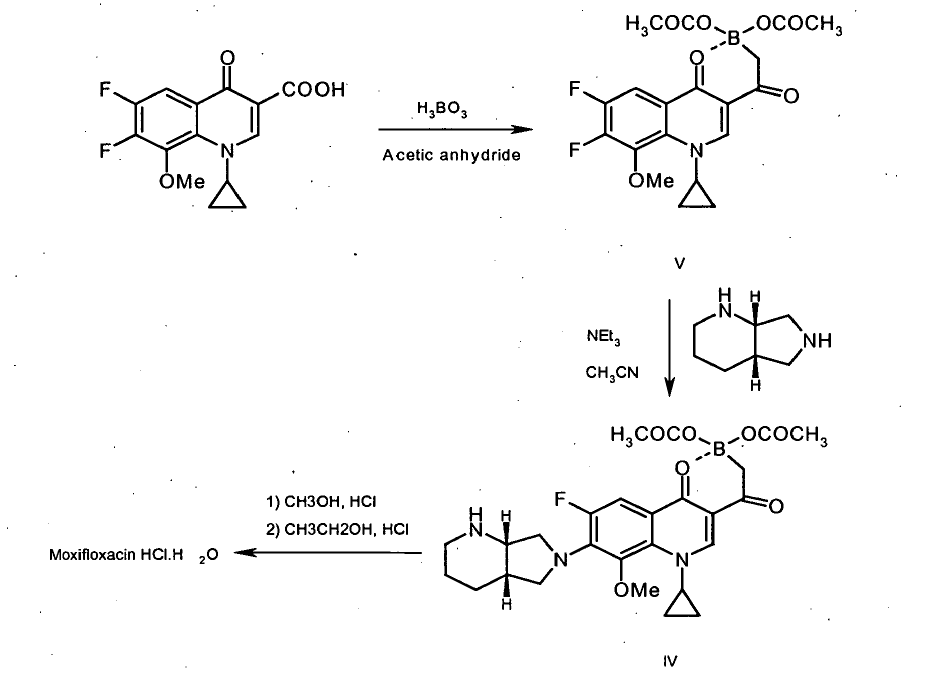

In this method the intermediates (IV) and (V) are isolated in separate operations, leading to losses at each stage, the overall yield being 56%.

The disadvantages of carrying out the synthesis in several steps are the low yields and the need to isolate the intermediate products, thereby hindering the production of moxifloxacin on an industrial scale.

### Object of the invention

The present invention relates to a "one pot" method for preparing moxifloxacin and its hydrochloride salt without need to isolate the intermediate compounds, the method comprises:
making a compound of formula (II) 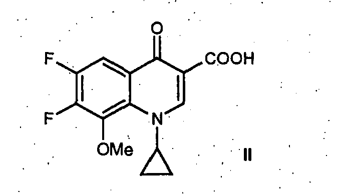
react with a silylating agent of formula ZSiR₃, in which Z is Me₃SiNH-or Cl-, and R is either a C₁₋₄ alkylic chain or phenyl, in an aprotic polar solvent, to provide the intermediate compound (VI), 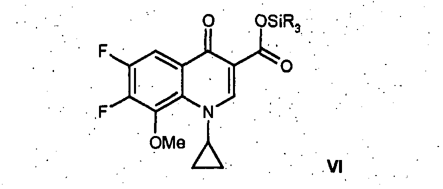 which without being isolated is then made to react with a boron trifluoride compound to obtain the intermediate compound (VII) 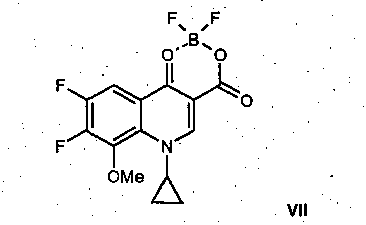 which is not isolated and is then made to react with (S,S)-2,8-diazabicyclo[4.3.0]nonane (III), 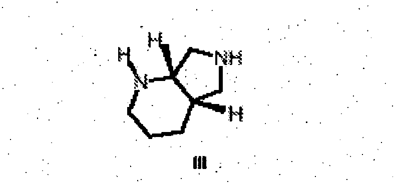 after adjusting the pH to a valued between 8 and 9, providing the intermediate compound (VIII) 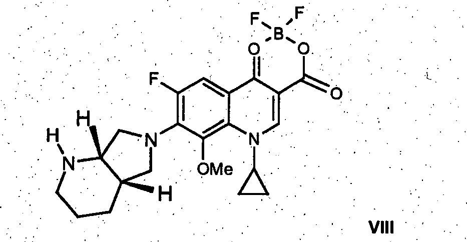 which, without being isolated, is made to react with a C₁₋₄ chain alcohol to yield moxifloxacin, and, if desired the hydrochloride salt of moxifloxacin can be obtained, with a content of less than 0.1% of the byproduct resulting from demethylation of the methoxy group on the 8-position of the ring.

Procedures termed "one pot" are characterised in that the synthesis is carried out in a single reaction vessel, without isolating the intermediate compounds and by means of successive addition of the reacting compounds, which means that the procedure can be carried out continuously.

The authors of the present invention have developed a new and more advantageous method than those so far described. The new method permits the end product moxifloxacin to be obtained on a large scale with a good yield and in pure form by using a "one pot" method in which a smaller amount of the high-cost reagent bicyclic amine is used. Moreover, the present method avoids the times and costs arising from isolating the intermediates and substantially avoids obtaining the impurity that comes from demethylation in 8-position of the ring.

### Detailed description of the invention

The method object of the invention consists in making a compound of formula (II) react with a silylating agent of formula ZSiR₃, in which Z is Me₃SiNH- or Cl-, and R is either a C₁₋₄ alkylic chain or phenyl, in an aprotic polar solvent, to provide the intermediate compound (VI), which without being isolated is then made to react with a boron trifluoride compound to obtain the intermediate compound (VII) which is not isolated and is then made to react with (S,S)-2,8-diaza-bicyclo[4.3.0]nonane (III), after adjusting the pH to a valued between 8 and 9, providing the intermediate compound (VIII) which, without being isolated, is made to react with a C₁₋₄ chain alcohol to yield moxifloxacin. Later, if desired, the hydrochloride salt of moxifloxacin can be obtained.

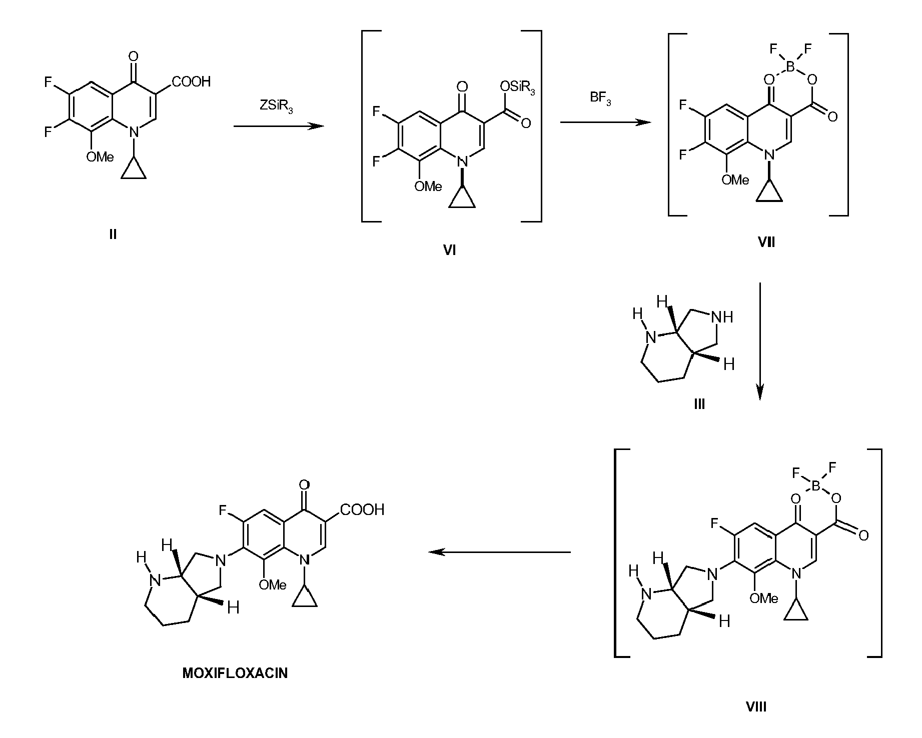

The starting compounds (II) and (III) can be prepared in accordance with methods already described in the literature, as for example in the patent EP-A-241206 and EP-A-550903 documents of, respectively.

The process of the invention starts with reaction of the compound (II) with a silylating agent. The silylating agent of formula ZSiR₃, in which Z is Me₃SiNH- or Cl-, and R is either a C₁₋₄ alkylic chain or phenyl, is a compound capable of converting the hydroxyl group of a carboxylic acid into a trialkyl, triarylsilyl or triarylalkylsilyl group in order to protect it or to activate it for subsequent reactions. Among suitable known silylating agents are those selected from the group containing hexamethyldisilazane (HMDS), trimethylsilyl chloride, tert-butyldimethylsilyl chloride, isopropyldimethylsilyl chloride, di-tert-butylmethylsilyl chloride, phenyldimethylsilyl chloride, triisopropylsilyl chloride. Preferably, the silylating agent used is hexamethyldisilazane (HMDS).

This stage is carried out in an aprotic polar solvent selected from acetonitryl, dimethylsulphoxide or dimethylformamide. Preferably, the aprotic polar solvent used is acetonitryl. A suitable reaction temperature lies within a range of 70° to 85°C, preferably between 78° and 82°C, for a time of between 1 and 2 hours, preferably 1 hour.

The intermediate compound (VI) obtained, which is not isolated, is made to react with a boron trifluoride compound, which is a compound capable of forming chelates between oxygen atoms, and one of the compounds used can be selected from the group containing boron trifluoride, complex of boron trifluoride or diethyl ether (boron trifluoride ethyletherate: BF₃.Et₂O), complex of boron trifluoride and tetrahydrofuran, tetrafluoroboric acid, complex of tetrafluoroboric acid and dimethyl ether. Preferably, the compound of boron trifluoride used is the complex of boron trifluoride ethyletherate.

This reaction is carried out in an aprotic polar solvent at a temperature lower than 15°C until complete disappearance of the compound (VI) is observed. At this point the pH of the mixture is adjusted with a base to a value ranging from 8 to 9.

The base lends the water an alkaline pH, and can be either inorganic or organic. Among the inorganic bases are the alkaline or alkaline earth bicarbonates and carbonates. The organic bases include tertiary amines such as triethylamine, tributylamine, 4-methylmorpholine, 4-dimethylaminopyridine or diisopropylethylamine. Preferably, the amine used is triethylamine.

The intermediate compound (VII) obtained, which is not isolated, reacts with the bicyclic amine (S,S)-2,8-diaza-bicyclo[4.3.0]nonane (III) in the aprotic polar solvent, while maintaining the temperature in the range of approximately 15-25°C for 5-6 hours, until complete conversion of the compound (VII) is observed.

This is followed by low-pressure distillation until a stirrable paste is obtained. To this paste is added the C₁₋₄ alkylic alcohol, in to remove the boron chelate at a temperature between 60° and 70°C, for the time necessary to achieve complete reaction of the compound (VIII), approximately 5-6 hours. The C₁₋₄ alkylic alcohol can be selected from the group of compounds such as methanol, ethanol, isopropanol, 1-propanol, 1-butanol, isobutanol, sec-butanol and tert-butanol. Preferably, the alcohol used in the reaction is methanol.

Once the boron chelate elimination reaction has been completed, the alcohol is evaporated off, water is added and the pH is adjusted firstly to between 11 and 12, and afterwards to a pH between 8 and 8.5. Finally, by means of extraction and concentration or precipitation and drying, the product moxifloxacin is obtained. Alternatively, it can be cooled and filtered, thereby providing moxifloxacin fluorhydrate.

Later, if desired, moxifloxacin hydrochloride can be yielded by adding hydrochloric acid.

Surprisingly, a new method has been found that permits moxifloxacin and moxifloxacin hydrochloride to be obtained from the precursor compound (II) with a yield greater than 80%, using smaller amounts of bicyclic amine, without need to isolate the intermediates involved in the various synthesis stages, of a purity and quality suitable for preparing pharmaceutical formulations, and with a content of less than 0.1% of the by-product resulting from demethylation of the methoxy group on 8-position of the ring.

### Example 1: Obtaining Moxifloxacin

In a four-mouth 500 ml flask with refrigerant, thermometer, mechanical stirring and nitrogen bubbler with the outlet connected to a washing flask with a solution of H₂SO₄ is placed 50 g (0.1694 moles, 1 eq) of the compound II, and 150 ml of acetonitryl (3 volumes), and it is heated to T = 76-80 °C (reflux). Once reflux has been reached, and maintaining T = 76-80 °C, a compensated addition funnel is used to add 19.14 g (0.1186 moles, 0.7 eq) of hexamethyldisilazane. Once the addition has been completed, the reaction is maintained with stirring at T = 76-80 °C for 1 hour. Once that period has elapsed the reaction mixture is cooled to T = 0-15 °C and 28.85 g (0.2033 moles, 1.2 eq) of boron trifluoride etherate is added, maintaining T<15 °C. Once the addition has been completed, it is maintained at T = 0-15°C until the starting product has disappeared (approximately 2 hours) and it is then adjusted to pH = 8-9 with triethylamine (approximately 30 ml). To the resulting suspension is added a solution of 32.07 g (0.2541 moles, 1.5 eq) of (S,S)-2,8-diazabicyclo[4.3.0]nonane and 25.71 g of triethylamine (0.2541 moles, 1.5 eq) in 150 ml of acetonitryl, maintaining T = 15-25 °C. The resulting amber solution is maintained with stirring under those conditions until the starting product has disappeared (approximately 5-6 hours). Once the reaction is completed it is distilled until a stirrable paste is obtained, 250 ml of methanol is added, the resulting solution is brought to T = 63-67 °C (reflux) and is maintained under those conditions until the boron compound (VIII) has disappeared (approximately 5-6 hours). The reaction completed, the methanol is distilled at low pressure, and to the resulting concentrate is added 250 ml of water. The resulting suspension is taken to pH = 12.0 with NaOH 30% and, later to pH=8.0-8.2 with HCl 35% and is extracted with methylene chloride (3 x 125 ml). The combined organic extracts are dried over anhydrous MgSO₄ and are concentrated to dryness, obtaining 60.72 g of base Moxifloxacin. Yield: 89.3%.

### Example 2: Obtaining Moxifloxacin

In a four-mouth 500 ml flask with refrigerant, thermometer, mechanical stirring and nitrogen bubbler with the outlet connected to a washing flask with a solution of H₂SO₄ is placed 50 g (0.1694 moles, 1 eq) of the compound II, 150 ml of acetonitryl (3 volumes), and it is heated to T = 76-80 °C (reflux). Once reflux has been reached, and maintaining T = 76-80 °C, a compensated addition funnel is used to add 19.14 g (0.1186 moles, 0.7 eq) of hexamethyldisilazane. Once addition has been completed, the reaction is maintained with stirring at T = 76-80 °C for 1 hour. Once that period has elapsed the reaction mixture is cooled to T = 0-15 °C and 28.85 g (0.2033 moles, 1.2 eq) of boron trifluoride etherate is added, maintaining T<15 °C. Once the addition has been completed, it is maintained at T = 0-15 °C until the starting product has disappeared (approximately 2 hours), and it is then adjusted to pH = 8-9 with triethylamine (approximately 30 ml). To the resulting suspension is added a solution of 32.07 g (0.2541 moles, 1.5 eq) of (S,S)-2,8-diazabicyclo[4.3.0]nonane and 25.71 g of triethylamine (0.2541 moles, 1.5 eq) in 150 ml of acetonitryl and maintaining T at 15-25°C. The resulting amber solution is maintained with stirring under those conditions until the starting product has disappeared (approximately 5-6 hours). Once the reaction is completed it is distilled until a stirrable paste is obtained, 250 ml of methanol is added, the resulting solution is brought to T = 63-67 °C (reflux) and is maintained under those conditions until the boron complex (compound VIII) has disappeared, (approximately 5-6 hours). The reaction completed, the methanol is distilled at low pressure and to the resulting concentrate is added 340 ml of water. The resulting suspension is taken to pH = 12 with NaOH 30% and then to pH = 8.0-8.2 with HCl 35%. The dark solution obtained is heated to T = 50-60 °C and is maintained under those conditions until the product has precipitated. Once the product has precipitated out, it is maintained under those conditions for 2 hours, following which it is cooled to T = 40 °C and filtered. The solid obtained is washed with 10 ml of water and dried at 40°C, to provide 48.52 g of base Moxifloxacin as a yellow solid.

### Example 3: Obtaining Moxifloxacin fluorhydrate

In a four-mouth 500 ml flask with refrigerant, thermometer, mechanical stirring and nitrogen bubbler with the outlet connected to a washing flask with a solution of H₂SO₄ is placed 50 g (0.1694 moles, 1 eq) of the compound II, 150 ml of acetonitryl (3 volumes), and it is heated to T = 76-80 °C (reflux). Once reflux has been reached, and maintaining T = 76-80 °C, a compensated addition funnel is used to add 19.14 g (0.1186 moles, 0.7 eq) of hexamethyldisilazane. Once addition has been completed, the reaction is maintained with stirring at T = 76-80 °C for 1 hour. Once that period has elapsed the reaction mixture is cooled to T = 0-15 °C and 28.85 g (0.2033 moles, 1.2 eq) of boron trifluoride etherate is added, maintaining T<15 °C. Once the addition has been completed, it is maintained at T = 0-15 °C until the starting product has disappeared (approximately 2 hours), and it is adjusted to pH = 8-9 with triethylamine (approximately 30 ml). To the resulting suspension is added a solution of 32.07 g (0.2541 moles, 1.5 eq) of (S,S)-2,8-diazabicyclo[4.3.0]nonane and 25.71 g of triethylamine (0.2541 moles, 1.5 eq) in 150 ml of acetonitryl while maintaining T = 15-25 °C. The resulting amber solution is maintained with stirring under those conditions until the starting product has disappeared (approximately 5-6 hours). Once the reaction is completed it is distilled until a stirrable paste is obtained, 250 ml of methanol is added, the resulting solution is brought to T = 63-67 °C (reflux) and maintained under those conditions until the boron complex has disappeared (approximately 5-6 hours). The reaction completed, the methanol is distilled at low pressure, and to the resulting concentrate is added 100 ml of methanol. The resulting suspension is cooled over a bath of water/ice (T = 0-5 °C) for 4 hours and filtered. The solid obtained is washed with 50 ml of methanol and dried at 40 °C in vacuum stove to constant weight. This provides 48.08 g of Moxifloxacin fluorhydrate. Yield: 67.60.

### Example 4: Obtaining Moxifloxacin hydrochloride

In a four-mouth 500 ml flask with refrigerant, thermometer, mechanical stirring and nitrogen bubbler with the outlet connected to a washing flask with a solution of H₂SO₄ is placed 50 g (0.1694 moles, 1 eq) of the compound (II), 150 ml of acetonitryl (3 volumes), and this is heated to T = 76-80 °C (reflux). Once reflux has been reached, and maintaining T = 76-80 °C, a compensated addition funnel is used to add 19.14 g (0.1186 moles, 0.7 eq) of hexamethyldisilazane. Once addition has been completed, the reaction is maintained with stirring at T = 76-80 °C for 1 hour. Once that period has elapsed the reaction mixture is cooled to T = 0-15 °C and 28.85 g (0.2033 moles, 1.2 eq) of Boron trifluoride etherate is added, maintaining T<15 °C. Once the addition has been completed, it is maintained at T = 0-15 °C until the starting product has disappeared (approximately 2 hours) and it is adjusted pH = 8-9 with triethylamine (approximately 30 ml). To the resulting suspension is added a solution of 32.07 g (0.2541 moles, 1.5 eq) of (S,S)-2,8-diazabicyclo[4.3.0]nonane and 25.71 g of triethylamine (0.2541 moles, 1.5 eq) in 150 ml of acetonitryl, maintaining T = 15-25 °C. The resulting amber solution is maintained with stirring under those conditions until the starting product has disappeared (approximately 5-6 hours). Once the reaction is completed it is distilled until a stirrable paste is obtained, 250 ml of methanol is added, the resulting solution is brought to T = 63-67 °C (reflux) and is maintained under those conditions until the boron compound (VIII) has disappeared, (approximately 5-6 hours). The reaction completed, the methanol is distilled at low pressure and to the resulting concentrate is added 340 ml of water. The resulting suspension is taken to pH = 12 with NaOH 30% and then to pH = 8.0-8.2 with HCl 35%. The dark solution obtained is extracted with benzyl alcohol (3 x 100 ml). The combined organic extracts are washed with 200 ml of water, and to the resulting organic phase is added 72.54 g of solution of hydrogen chloride in ethyl alcohol 8.52% (6.18 g, 0.1694 moles, 1 eq). 1320 ml of toluene is then added to the solution obtained, producing precipitation of Moxifloxacin·HCl as a yellow solid. The resulting suspension is maintained with stirring at ambient temperature for one hour, following which it is filtered and the solid is washed with toluene (2 x 70 ml). The moist product is dried at 40 °C to provide 59.82 g of Moxifloxacin·HCl. Yield: 80.6%.

## Claims

1. Method for preparing moxifloxacin, **characterized in that**, in a "one pot" reaction, the method comprises:
making a compound of formula (II) 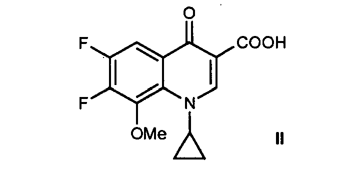
react with a silylating agent of formula ZSiR₃, in which Z is Me₃SiNH-or Cl-, and R is either a C₁₋₄ alkylic chain or phenyl, in an aprotic polar solvent, to provide the intermediate compound (VI), 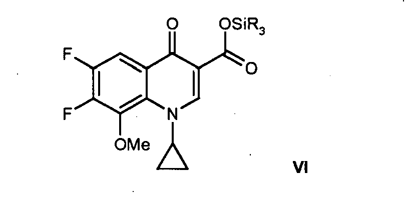
which without being isolated is then made to react with a boron trifluoride compound to obtain the intermediate compound (VII) 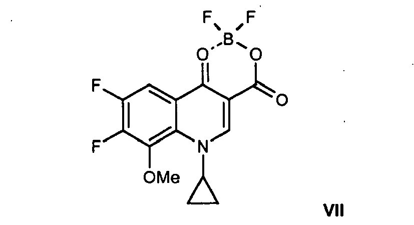
which is not isolated and is then made to react with (S,S)-2,8-diazabicyclo[4.3.0]nonane (III), 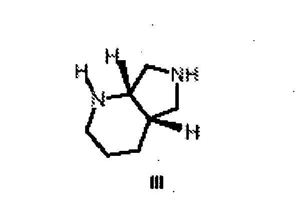 after adjusting the pH to a valued between 8 and 9, providing the intermediate compound (VIII) 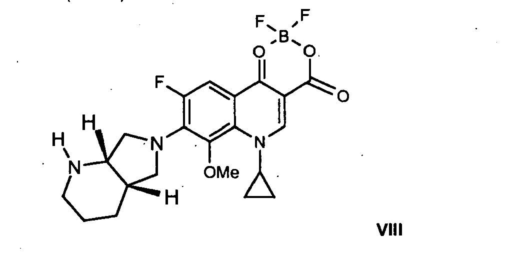
which, without being isolated, is made to react with a C₁₋₄ chain alcohol to yield moxifloxacin, and, if desired the hydrochloride salt of moxifloxacin can be obtained, with a content of less than 0.1% of the byproduct resulting from demethylation of the methoxy group on the 8-position of the ring.

2. Method according to claim 1, wherein said silylating agent is hexamethyldisilazane, said compound of boron trifluoride is the complex of boron trifluoride ethyletherate, said C₁₋₄ alcohol is methanol, said aprotic polar solvent is acetonitryl and said base is a tertiary amine.

3. Method according to claim 2, wherein said tertiary amine is triethylamine.

## Patentansprüche

1. Verfahren zur Herstellung von Moxifloxacin, **dadurch gekennzeichnet, dass**, in einer "Eintopf"-Reaktion, das Verfahren umfasst:
Herstellen einer Verbindung der Formel (II) 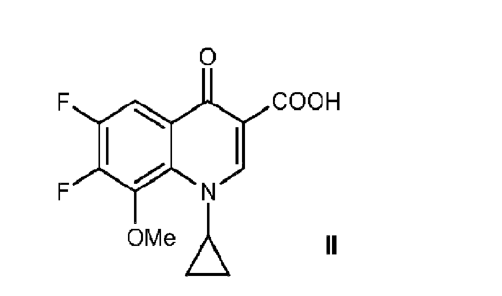
Umsetzen mit einem Silylierungsmittel der Formel ZSiR₃, in der Z Me₃SiNH- oder Cl- ist und R entweder eine C₁₋₄ Alkylkette oder Phenyl ist, in einem aprotischen polaren Lösungsmittel, um eine Zwischenverbindung (VI) bereitzustellen, 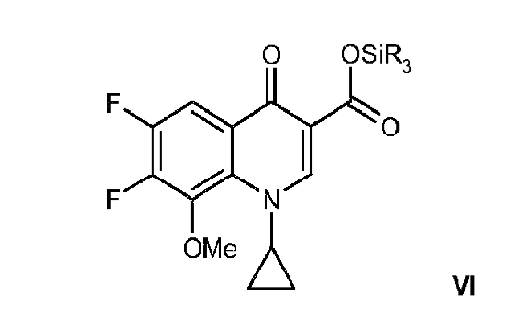 die, ohne isoliert zu werden, dann umgesetzt wird mit einer Bortrifluoridverbindung, um eine Zwischenverbindung (VII) zu erhalten 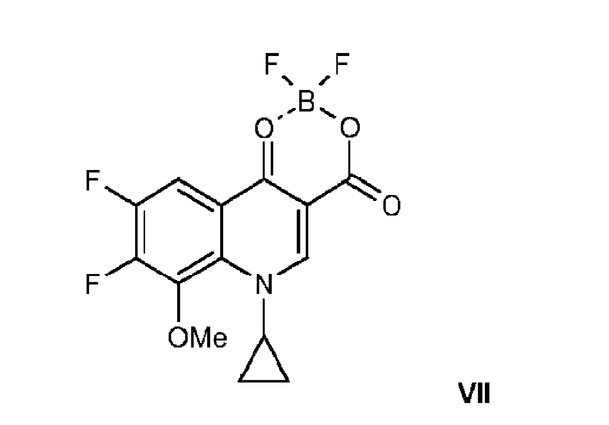 die nicht isoliert wird und dann umgesetzt wird mit (S,S)-2,8-Diazabicyclo[4.3.0]nonan (III), 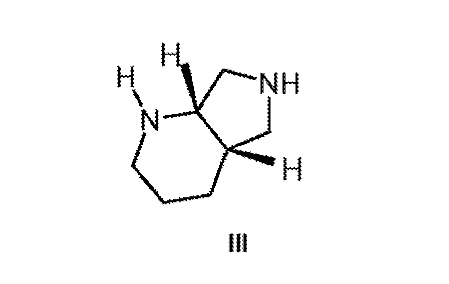 nach Einstellen des pH auf einen Wert von 8 bis 9, Bereitstellen der Zwischenverbindung (VIII), 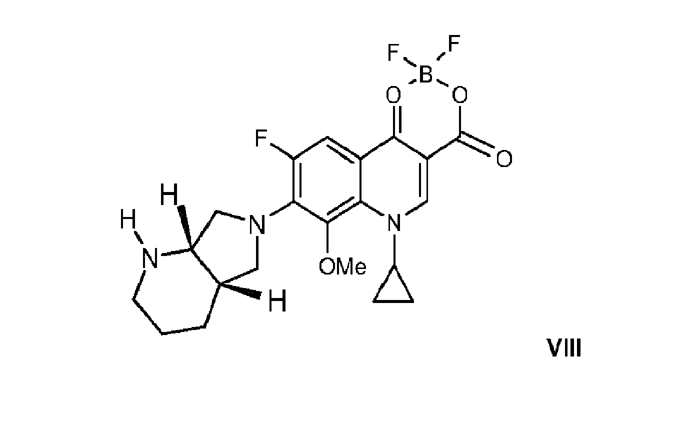 die, ohne isoliert zu werden, mit einem C₁₋₄ kettigen Alkohol umgesetzt wird, um Moxifloxacin zu erhalten, und, falls gewünscht, das Hydrochloridsalz von Moxifloxacin erhalten werden kann, mit einem Gehalt von unter 0,1% des Nebenprodukts, welches aus der Demethylierung der Methoxygruppe an der 8-Position des Rings resultiert.

2. Verfahren nach Anspruch 1, wobei das Silylierungsmittel Hexamethyldisilazan ist, die Bortrifluoridverbindung der Komplex aus Bortrifluoridethyletherat ist, der C₁₋₄ Alkohol Methanol ist, das aprotische polare Lösungsmittel Acetonitryl ist und die Base ein tertiäres Amin ist.

3. Verfahren nach Anspruch 2, wobei das tertiäre Amin Triethylamin ist.

## Revendications

1. Procédé de préparation de moxifloxacine, **caractérisé en ce que**, au cours d'une réaction « one pot », ledit procédé consiste à :
faire réagir un composé correspondant à la formule (II) 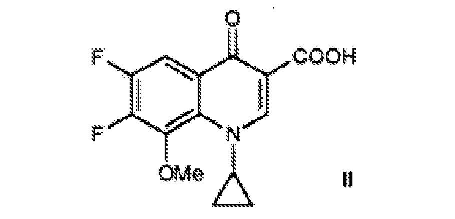
avec un agent de silylation correspondant à la formule ZSiR₃, où Z représente Me₃SiNH- ou Cl- et R représente une chaîne alkyle C₁₋₄ ou phényle, dans un solvant polaire aprotique afin de fournir le composé intermédiaire (VI), 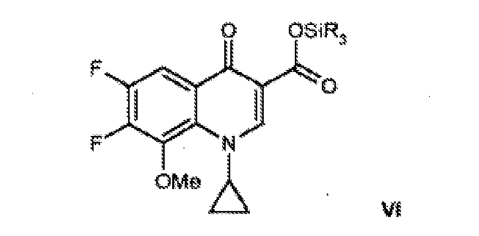
que l'on fait ensuite réagir, sans l'avoir isolé, avec un composé de trifluorure de bore afin d'obtenir le composé intermédiaire (VII) 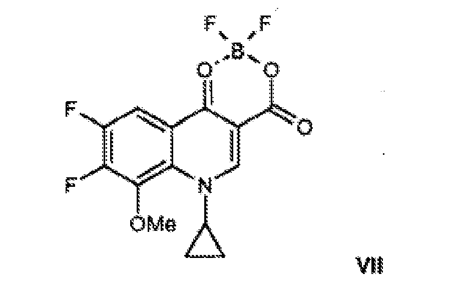
qui n'est pas isolé et que l'on fait ensuite réagir avec du (S,S)-2,8-diazabicyclo[4.3.0]nonane (III) 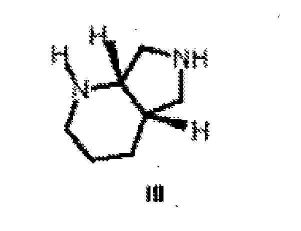
après avoir ajusté le pH à une valeur entre 8 et 9, ce qui donne le composé intermédiaire (VIII) 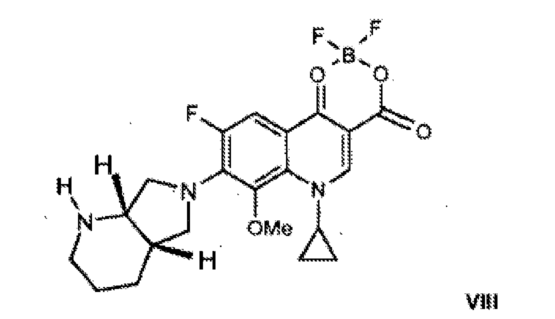
que l'on fait réagir, sans l'avoir isolé, avec un alcool à chaîne C₁₋₄ afin de produire de la moxifloxacine et, si on le souhaite, le sel d'hydrochlorure de moxifloxacine peut être obtenu, ceci avec une teneur de moins de 0,1 % en sous-produits résultant de la déméthylation du groupe méthoxy sur la position 8 du cycle.

2. Procédé selon la revendication 1, dans lequel l'agent de silylation consiste en du hexaméthyldisilazane, ledit composé de trifluorure de bore est le complexe éthyléthérate de trifluorure de bore, ledit alcool C₁₋₄ consiste en du méthanol, ledit solvant polaire aprotique consiste en de l'acétonitryle, et ladite base consiste en une amine tertiaire.

3. Procédé selon la revendication 2, dans lequel ladite amine tertiaire consiste en de la triéthylamine.
